Europäisches Patentamt

European Patent Office

Office européen des brevets

(11) Veröffentlichungsnummer: **0 400 341**
**A2**

## (12) EUROPÄISCHE PATENTANMELDUNG

(21) Anmeldenummer: 90108213.1

(22) Anmeldetag: 30.04.90

(51) Int. Cl.⁵: **A61B 17/22, A61B 6/04**

(30) Priorität: 01.06.89 DE 3917858

(43) Veröffentlichungstag der Anmeldung:
05.12.90 Patentblatt 90/49

(84) Benannte Vertragsstaaten:
**CH DE ES FR GB IT LI**

(71) Anmelder: DORNIER MEDIZINTECHNIK GMBH
Postfach 1128
D-8034 Germering 1(DE)

(72) Erfinder: Viebach, Thomas, Dipl.-Ing.
Ammerhof 2
D-8121 Pähl(DE)

(74) Vertreter: Landsmann, Ralf, Dipl.-Ing.
DORNIER GMBH - Patentabteilung - Kleeweg
3
D-7990 Friedrichshafen 1(DE)

(54) Ankoppelfenster für einen Lithotripter.

(57) Ankoppelfenster (AF) zur Einleitung von Stosswellen in einen Patientenkörper (PK). Durch Verwendung eines formsteifen Materials ergeben sich Vorteile für die Patientensicherheit, den Regelungsaufwand und den Behandlungskomfort bei Lithotriptern.

# Fig. 1

EP 0 400 341 A2

## Ankoppelfenster für einen Lithotripter

Die Erfindung betrifft ein Ankoppelfenster für die Behandlung mit extrakorporal erzeugten Stosswellen, insbesondere für einen Lithotripter, nach dem Oberbegriff des Anspruchs 1.

Bei Lithotriptoren wurde bisher die Stosswellenankopplung über ein offenes Wasserbad oder ein mit einer flexiblen Membran abgeschlossenes Wasserkissen realisiert. Die Vorteile des geschlossenen Wasserkissens in der klinischen Praxis sind unumstritten, jedoch ergeben sich auch Nachteile, wie z.B. die aufwendige Druckregelung, eine unbeabsichtigte Patientenverschiebung beim Ankoppeln und Positionieren, eine Faltenbildung des Kissens oder Kollisionsprobleme.

Aus der **DE-PS 35 32 678** ist eine Liege mit einem Fenster mit Wasser gefüllter Vorlaufstrecke mit oder ohne Membranabdeckung bekannt. Auch dabei kann eine Druckänderung im Wasser zu einer unbeabsichtigten Patientenverschiebung führen.

Aufgabe der Erfindung ist es, ein Stosswellenankoppelfenster vorzuschlagen, bei der eine unbeabsichtigte Patientenberührung oder -verschiebung beim Positionieren nicht erfolgt.

Diese Aufgabe wird erfindungsgemäss gelöst von einem Ankoppelfenster mit den Merkmalen des Anspruchs 1. Ausführungen der Erfindung sind Gegenstände von Unteransprüchen.

Die Erfindung beruht darauf, die Koppelfläche zwischen dem Patienten und der Stosswellenquelle oder dem Ankoppelmedium steif auszubilden, insbesonders durch die Materialauswahl. Steif im Sinne der Erfindung ist ein Fenster, das beim Auflegen des Patienten nicht oder nur unwesentlich seine Form verändert (formstabil, starr).

Die Form des Ankoppelfensters in einer entsprechenden Patientenliege ist im wesentlichen in Längsrichtung eben und quer dazu konkav (also einer Rinne entsprechend).

In einer anderen Ausführung kann der Bereich des Ankoppelfensters in Patientenlängsrichtung konvex und in Patientenquerrichtung konkav sein, das heisst es kann sattelförmig ausgebildet sein. Bei kleineren Ankoppelflächen sind auch ganz ebene oder in beiden Richtungen konvex gekrümmte Geometrien möglich.

Die Stosswelleneinheit befindet sich regelmässig unter diesem Koppelfenster, z.B. in einem Wasserbad. Ein freibeweglicher Therapiekopf ist z.B. durch ein konvex gekrümmtes Ankoppelfenster abgeschlossen. Die Stosswellenquelle ist im Therapiekopf zur freien Positionierung beweglich aufgehängt.

Folgende Vorteile ergeben sich durch die Erfindung:

- Der Patient liegt bequem, ohne die Gefahr des Einsinkens und ohne drückende Liegenkanten oder Therapiekopfränder.
- Der Patient spürt absolut nichts von den beim Positionieren auftretenden Relativbewegungen zwischen ihm und der Stosswellenquelle
- Beim Positionieren treten keine Kräfte auf, die den Patienten und den Stein verlagern könnten.
- Die Stosswelleneinheit kann jederzeit (z.B. für die Röntgenortung) weggefahren und anschliessend an den Ausgangspunkt zurückgefahren werden, ohne die eingestellte Steinlage durch An- und Abkoppeln zu verändern.
- Für die Patienten besteht keinerlei Gefährdung durch Kollision mit der Stosswelleneinheit oder mit einem dort befestigten Ultraschall- oder Röntgeneinrichtungen.
- Eine genaue Druckregelung ist überflüssig.

Die Ausbildung des Ankoppelfensters ist so, dass alle Patienten darauf über längere Zeit liegen können und gleichzeitig ein grossflächiger Hautkontakt gewährleistet ist. Der Körperkontakt kann ausser durch Koppelgel durch Zuhilfenahme von gallertähnlichen Massen (z.B. sogenannten Ultraschallvorlaufstrecken wie Mitteln, die unter den Bezeichnungen: Sonar-aid oder Reston im Handel sind) verbessert werden.

Auswahlkriterien für das Material sind unter anderem die akustische Impedanz, die Röntgendurchlässigkeit, die mechanische Steifigkeit und die Verarbeitbarkeit. Polyäthylen ist in diesen vier Punkten gut geeignet.

Die Stosswellenquelle kann sich entweder in einem Wasserbad unter dem Fenster bewegen oder sie ist beweglich in einem Topf aufgehängt, dessen oberer Rand am Fenster abdichtet. Dieser Topf ist dann unter dem Fenster verschiebbar aufgehängt.

Die Erfindung wird anhand von vier Figuren näher beschrieben.
Es zeigen:

Fig. 1 eine erfindungsgemässe Liege.

Fig. 2 zwei Möglichkeiten der Ankoppelung des Patienten,

Fig. 3 zwei Möglichkeiten der Anbringung einer Stosswellenquelle,

Fig. 4 einen erfindungsgemässen Therapiekopf.

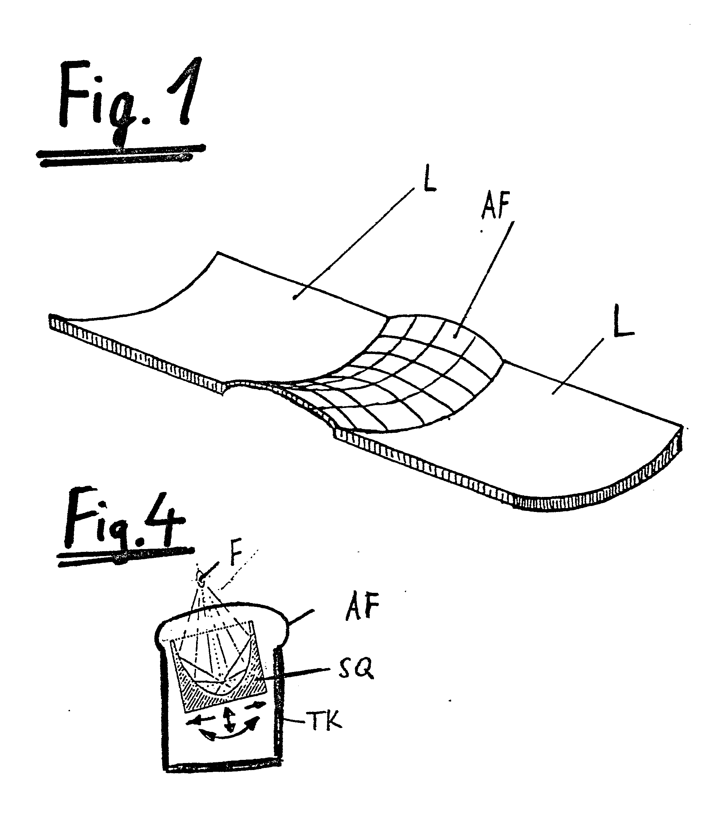

Fig. 1 zeigt eine Patientenliege, bestehend aus den Liegeflächen L und dem Ankoppelfenster AF. Das Koppelfenster AF ist hier dreidimensional gekrümmt, sattelförmig ausgebildet. Die Krümmung in Patientenlängsrichtung ist konvex, in Patientenquerrichtung konkav. Die Liegeflächen L sind ebenfalls konkav gekrümmt.

Das Koppelfenster AF kann z.B. durch Tiefziehen einer Thermoplastplatte hergestellt sein.

Fig. 2 zeigt links einen Patientenkörper PK auf dem Koppelfenster AF in Rückenlage (Wirbelsäule und Nieren eingezeichnet). Da das Koppelfenster AF erfindungsgemäss starr ist, sind Flächen vorhanden, an denen sich der Patientenkörper PK nicht an das Koppelfenster AF anschmiegt. Diese Flächen sind hier mit einem Koppelgel (dunkel gezeichnet) gefüllt.

Fig. 2 zeigt rechts einen Patientenkörper PK in Bauchlage auf dem Koppelfenster AF. Da sich der Bauch des Patienten besser an das Koppelfenster AF an schmiegt, ist hier weniger Koppelgel erforderlichen.

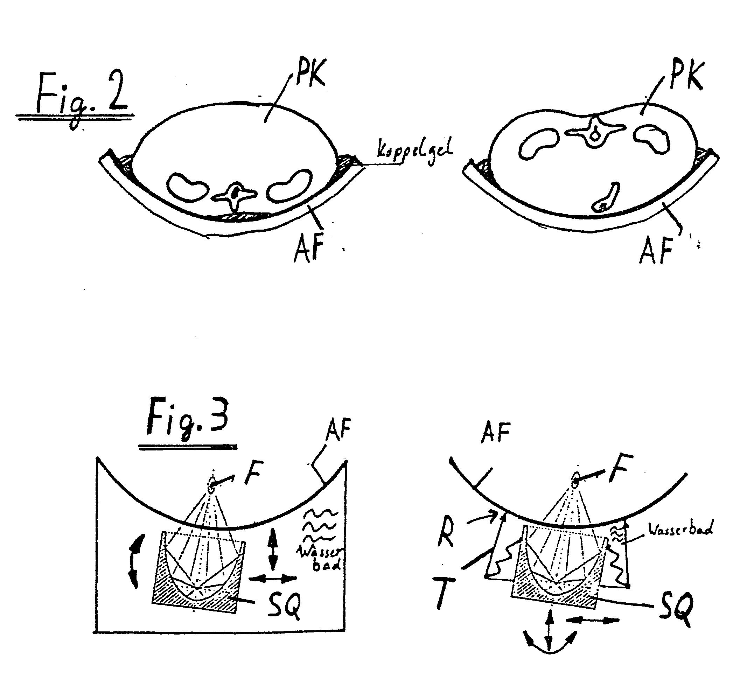

Fig. 3 zeigt zwei Varianten der Anbindung einer Stosswellenquelle SQ an das Koppelfenster AF. Links befindet sich die fokussierende Stosswellenquelle SQ in einem Wasserbad unter der Koppelfläche. Eingezeichnet ist die Stosswellenquelle SQ mit ihrem Brennpunkt F, in dem sich das zu zerstörende Konkrement befindet. Die Stosswellenquelle besitzt zur Positionerung mehrere Freitheitsgrade der Bewegung und Rotation, wie eingezeichnet.

In Fig. 3 ist rechts eine weitere Ausführung gezeigt, bei der die Stosswellenquelle SQ in einem Topf T unter der Koppelfläche K aufgehangen ist. Das Wasserbad zur Ankopplung wird dadurch wesentlich kleiner. Der obere Rand des Topfes T dichtet and er Koppelfläche K ab. Der Topf kann verschiebbar sein.

Fig. 4 zeigt einen Therapiekopf TK mit starrer Stosswellenankopplung. Die Stosswellenquelle SQ ist wieder in einem topfähnlichen Therapiekopf TK beweglich aufgehängt. Deshalb sind nach der Ankopplung des Therapiekopfes TK an den Patienten zur Positionerung der Stosswellenquelle auf den Stein (im Brennpunkt) keine Relativbewegungen des Ankoppelfensters AF gegenüber dem Patienten erforderlich. Die anderen Auflageflächen für den Patientenkörper sind hier nicht gezeigt.

Ansprüche

1. Ankoppelfenster (AF) zur Ankoppelung von Stosswellenerzeugern an einen Patientenkörper (PK), insbesonders zum Abschluss von Liegenfenstern bei Lithotriptern mit unter der Liege (L) angeordneter Stosswellenquelle (SQ) oder zum Abschluss eines Therapiekopfes (TK) bei Lithotriptern mit unabhängig von der Liege (L) angeordneten Stosswellenerzeugern, **dadurch gekennzeichnet,** dass das Ankoppelfenster (AF) steif ist.

2. Ankoppelfenster nach Anspruch 1 in einer Patientenliege, **dadurch gekennzeichnet,** dass das Koppelfenster (AF) in Patientenlängsrichtung eben und quer dazu konkav ist.

3. Ankoppelfenster nach Anspruch 1 in einer Patientenliege, **dadurch gekennzeichnet,** dass es in Patientenlängsrichtung konvex und quer dazu konkav ist.

4. Ankoppelfenster nach Anspruch 1 in einer Patientenliege, **dadurch gekennzeichnet,** dass es in beiden Richtungen eben oder konvex ist.

5. Ankoppelfenster nach Anspruch 1 nach einem Therapiekopf (TK), **dadurch gekennzeichnet,** dass es eben oder konvex ist.

6. Ankoppelfenster nach einem der vorhergehenden Ansprüche, **dadurch gekennzeichnet,** dass es aus Polyäthylen besteht und eine im wesentlichen gleichmässige Dicke aufweist.

7. Ankoppelfenster nach einem der vorhergehenden Ansprüche, **dadurch gekennzeichnet,** dass es aus einem Kunststoff mit einer ähnlichen akustischen Impedanz besteht wie der des Wassers.

# Fig. 1

L
AF
L

# Fig. 4

F
AF
SQ
TK

Fig. 2

PK

Koppelgel

AF

PK

AF

Fig. 3

AF

F

Wasser bad

SQ

AF

F

R

T

Wasserbad

SQ